# EUROPEAN PATENT APPLICATION

(11) **EP 3 409 290 A1**
(43) Date of publication of application: **05.12.2018**
(21) Application number: 17744582.2
(22) Date of filing: 26.01.2017
(51) Int. Cl.: A61K 47/02, A61K 9/08, A61K 31/445

(54) **PURGATIVE COMPOSITION**

(30) Priority: 28.01.2016 KR 20160010395
(71) Applicant: CTC Bio, Inc., Hongcheon-gun, Gangwon-do 25142 (KR)
(72) Inventor: JEON, Hong Ryeol, Suwon-si Gyeonggi-do 16543 (KR); LEE, Bong-Sang, Suwon-si Gyeonggi-do 16501 (KR); KIM, Hyun-Il, Suwon-si Gyeonggi-do 16571 (KR); LEE, Han-Seung, Namyangju-si Gyeonggi-do 12103 (KR)
(74) Representative: Graf von Stosch Patentanwaltsgesellschaft mbH
(86) International application number: PCT/KR2017/000943
(87) International publication number: WO 2017/131461

(57) **Abstract**

The present invention relates to a purgative composition containing polyethylene glycol and sodium picosulfate, wherein the stability of sodium picosulfate is improved by adding a pH controller. According to the present invention, the purgative composition ensures the stability of medicinal ingredients and has a great advantage of having a small dose even while showing a bowel cleansing rate greater than or equal to a commercial preparation, which should be taken in a large dose. In addition, according to the present invention, the composition has high compliance due to a good taste.

## Description

### [Technical Field]

The present disclosure relates to purgative compositions comprising polyethylene glycol and sodium picosulfate. In particular, the present disclosure relates to purgative compositions having improved stability.

### [Background Art]

Purgative compositions are used for various purposes like X-ray examination, endoscopy, radiography, intestinal cleansing before surgery and so on.

Polyethylene glycol (PEG) is used as an effective agent of these purgative compositions, but compositions using polyethylene glycol have the inconvenience of taking a very large amount in a short time.

In order to alleviate this inconvenience, there is an attempt to reduce the dose of solvent (water) by administering polyethylene glycol together with a stimulant laxative. One of the stimulant laxatives is sodium picosulfate.

However, the pharmaceutical problems associated with laxative compositions containing polyethylene glycol and sodium picosulfate have not yet been elucidated.

### [Disclosure]

### [Technical Problem]

Accordingly, one object of the present disclosure is to provide a composition for identifying and solving various problems of a purgative composition comprising polyethylene glycol and sodium picosulfate.

### [Technical Solution]

To achieve the object, the present disclosure provides a purgative composition comprising polyethylene glycol and sodium picosulfate, wherein the composition further comprises a pH controller in order to improve the stability of sodium picosulfate.

The present inventors have found that polyethylene glycol has a very negative effect on the stability of sodium picosulfate in studying a composition containing both polyethylene glycol and sodium picosulfate, and completed the present invention by confirming that it can be solved by adding a pH controller.

In the present invention, "purgative" means a substance or composition that promotes defecation. Thus, purgatives include a category of diarrhea. For example, a purgative include causing mild catharsis, which causes diarrhea ("partial purgation") as well as stronger catharsis that completely ("complete purgation") or almost completely empties the colon.

Preferably, the present disclosure provides a purgative composition comprising polyethylene glycol and sodium picosulfate, further comprising an alkalizer as the pH controller to improve the stability of sodium picosulfate.

In the purgative composition of the present invention, the alkalizer is sodium hydroxide, sodium phosphate, sodium hydrogencarbonate, sodium citrate, potassium citrate, potassium hydroxide, potassium phosphate, magnesium carbonate, sodium carbonate, ammonium carbonate, or a mixture thereof. In particular, sodium hydrogencarbonate is more preferable when taking into account bowel cleansing rate, drug compliance based on improvement in taste, composition's stability which are main purpose of purgative compositions.

Preferably, the polyethylene glycol comprised in the purgative composition has 1,000 to 10,000 of number average molecular weight. When the molecular weight is less than 1,000, it can be easily absorbed by the intestinal mucosa and its role as a laxative may be insufficient. More preferably, a polyethylene glycol having a number average molecular weight of 2,000 to 6,000 may be used, and still more preferably a number average molecular weight of 3,000 or 4,800 may be used.

The parenteral compositions according to the present invention may be in the form of powders, granules, powders, suspensions, syrups, or solutions.

When the poultice composition according to the present invention is in the form of a liquid, the pH of the liquid composition is preferably 7 to 12 in view of improving the stability of sodium picosulfate.

When the poultice composition according to the present invention is a non-liquid formulation, the pH of the solution prepared by dissolving such a poultice composition in purified water to a total volume of 300 ml is preferably 7 to 12 in view of improving the stability of sodium picosulfate.

The purgative compositions according to the present invention may be in the form of powders, granules, powders, suspensions, syrups, or solutions.

When the purgative composition according to the present invention is in the form of a liquid, the pH of the liquid composition is preferably 7 to 12 in view of improving the stability of sodium picosulfate.

When the purgative composition according to the present invention is a non-liquid formulation, the pH of the solution prepared by dissolving such a purgative composition in purified water to a total volume of 300 ml is preferably 7 to 12 in view of improving the stability of sodium picosulfate.

The purgative composition according to the present invention may comprise, to the extent not to interfere with the purpose of the present invention, other osmotic-type laxative(s) other than polyethylene glycol, non-osmotic laxative(s) other than sodium picosulfate, solvent(s) (water, ethanol, etc.), suspending agent(s), viscosifying agent(s), sweetening agent(s), flavoring agent(s), coloring agent(s), and the like.

The osmotic-type laxative acts to increase intestinal osmotic pressure thereby promoting retention of intestinal fluid. Examples of the osmotic-type laxative which can be used in the purgative composition include, but are not limited to, magnesium citrate, magnesium chloride, magnesium hydroxide, magnesium phosphate, magnesium sulfate, magnesium tartrate, sodium phosphate, sodium tartrate, sodium sulfate, potassium tartrate, magnesium oxide, sodium sulfate, glycerin, sorbitol, mannitol, lactitol, sugar, L-sugar, lactulose, and the like.

The non-osmotic laxative may be not only stimulant laxatives that directly stimulate the nerve endings in the large intestine mucosa but also prokinetic laxatives that stimulate the motility of the gastrointestinal tract. Examples of the non-osmotic laxative which can be used in the purgative composition include, but are not limited to, mineral oil, aloe, bisacodyl, casanthranol, cascara, castol oil, dantron, dehydroholic acid, phenolphthalein, sennosides, docusate, bethanachol, colchicines, misoprostol, cisapride, norcisapride, paraffin, rhein, tegaserod, and the like.

The sweetening agent comprised in the purgative composition may be, but be not limited to, saccharin, saccharin sodium, xylitol, sorbitol, mannitol, maltitol, lactitol, isomalt, stevioside, erythritol, aspartame, acesulfame potassium, sucralose and the like which exhibit a sweetening effect in a small amount and quick solubility, as well as common saccharides such as glucose, sucrose, dextrose, fructose, maltose.

When the purgative composition according to the present invention is in the form of a liquid, the method for preparing the composition comprises the steps of (S1) putting polyethylene glycol in a container, adding adequate amount of water, and melting it by heating to about 50 to 80 °C (preferably about 60 °C), (S2) adding osmotic-type laxative(s), non-osmotic laxative(s), sweetening agent(s) and the like, and (S3) adjusting the volume with purified water, followed by completely dissolving them at about 70 to 90 °C(preferably about 80 °C).

In the present invention, the term "about" means ± 10% of the value.

More preferably, the purgative composition of the present invention comprises about 100-200 g of polyethylene glycol having a number average molecular weight of 3000-5000, about 40-70 g of sorbitol, about 0.005-0.03 g of sodium picosulphate, about 0.1-0.4 g of sodium hydrogencarbonate, and adequate amount of sweetening agent(s), which may be dissolved in purified water to give a total volume of about 300 ml.

The present invention also provides a liquid purgative composition comprising about 100-200 g of polyethylene glycol having a number average molecular weight of 3000-5000, about 40-70 g of sorbitol, about 0.005-0.03 g of sodium picosulfate, about 0.1-0.4 g of sodium hydrogencarbonate, adequate amount of sweetening agent(s) and residual amount of purified water in a total volume of 300 ml.

The purgative composition according to the present invention is very advantageous in that not only the stability, the main purpose according to the present invention, is secured but also the dosage is less than that of the commercial product which requires taking a large amount while exhibiting the same or better bowel cleansing rate. Also, the composition of the present invention has good taste and high drug compliance.

The present invention also provides a method for cleansing bowel using the purgative composition. More specifically, the cleansing method is carried out by administering 100 to 200 mL of the purgative composition and 300 to 600 mL of water, administering 400 to 600 mL of water within 30 minutes, and repeating the same one time (100 to 200 mL of the purgative composition and 300 to 600 mL of water, and further taking 400 to 600 mL of water within 30 minutes).

Preferably, 125 to 175 mL of the purgative composition and 350 to 550 mL of water are administered, and 450 to 550 mL of water is further administered within 30 minutes, and the same can be repeated once more. Preferably, 150 mL of the purgative composition and 500 mL of water are administered, and 500 mL of water is further administered within 20 minutes, and the same method may be repeated one more time.

Most preferably, 150 mL of the purgative composition and 350 mL of water are administered within 30 minutes, and further 500 mL of water is administered within 30 minutes. Thereafter, after at least 1 hour, the same method (150 mL of the purgative composition and 350 mL of water are administered within 30 minutes, and further 500 mL of water is administered within 30 minutes) is repeated one more time. The bowel cleansing method using the purgative composition of the present invention exhibits sufficient bowel cleansing effect even when the total amount of the solution taken is reduced to about 2 L.

Thus, the present disclosure provides a method for cleansing bowel, using a liquid purgative composition comprising 100-200 g of polyethylene glycol having a number average molecular weight of 3000-5000, 40-70 g of sorbitol, 0.005-0.03 g of sodium picosulfate, 0.1-0.4 g of alkalizer (preferably, sodium hydrogencarbonate), and a suitable amount of sweetener in 300 ml of solution, wherein the method comprises administering 100 to 200 ml of the purgative composition together with 300 to 600 ml of water, and then additional 400 to 600 ml of water within 30 minutes, and administering 100 to 200 ml of the purgative composition together with 300 to 600 ml of water and then additional 400 to 600 ml of water within 30 minutes. More preferably, the present disclosure provides the method wherein the method administering 150 ml of the purgative composition together with 350 ml of water within 30 minutes, and then additional 500 ml of water within 30 minutes, and after at least 1 hour, administering 150 ml of the purgative composition together with 350 ml of water within 30 minutes and then additional 500 ml of water within 30 minutes.

### [Advantageous Effects]

The purgative composition according to the present invention has a great advantage that the stability of the active ingredients is ensured and the required dosage is less than that of a commercial product which requires taking a large amount while showing a bowel cleansing rate greater than or equal to the commercial product. Also, the composition of the present invention has good taste and high drug compliance due to a good taste.

### [Mode for Invention]

Hereinafter, the present disclosure is described in considerable detail with examples to help those skilled in the art understand the present disclosure. However, the following examples are offered by way of illustration and are not intended to limit the scope of the invention. It is apparent that various changes may be made without departing from the spirit and scope of the invention or sacrificing all of its material advantages.

### Experimental Example 1

0.01g of sodium picosulfate, 150g of polyethylene glycol 4000, and 54.6g of sorbitol were dissolved with heat in purified water according to the following tables and the total volume was 300 ml. The solution was stored at room temperature, in accelerated condition (40°C, 75%RH), and stress condition (60°C, 75%RH). The content of sodium picosulfate was evaluated. The content of sodium picosulfate was determined by 'Sodium picosulfate' test method of *British Pharmacopoeia* using high performance liquid chromatography. The results were shown in Tables 1 to 3 below.

**[Table 1]**

| Content (%) | Initial | After 1 week Room temp. | After 2 weeks Room temp. |
|---|---|---|---|
| sodium picosulfate | 102.4 | 102.2 | 102.4 |
| sodium picosulfate + polyethylene glycol 4000 | 101.7 | 101.6 | 101.1 |
| sodium picosulfate + Sorbitol | 101.9 | 101.7 | 102.0 |

**[Table 2]**

| Content (%) | Initial | After 1 week Accelerated condition | After 2 weeks Accelerated condition |
|---|---|---|---|
| sodium picosulfate | 102.4 | 102.2 | 103.6 |
| sodium picosulfate + polyethylene glycol 4000 | 101.7 | 101.3 | **95.5** |
| sodium picosulfate + Sorbitol | 101.9 | 102.0 | 103.1 |

**[Table 3]**

| Content (%) | Initial | After 1 week Stress condition | After 2 weeks Stress condition |
|---|---|---|---|
| sodium picosulfate | 102.4 | 102.4 | 102.5 |
| sodium picosulfate + polyethylene glycol 4000 | 101.7 | **75.1** | **6.7** |
| sodium picosulfate + Sorbitol | 101.9 | 102.2 | 103.0 |

As shown in Tables 1 to 3 above, sodium picosulfate was very unstable when mixed with polyethylene glycol.

### Experimental Example 2

0.01g of sodium picosulfate, 150g of polyethylene glycol 3350, 54.6g of sorbitol, and the following amount of sodium hydrogencarbonate or sodium citrate were dissolved with heat in purified water and the total volume was 300 ml. The solution was stored at room temperature or stress condition (60°C, 75%RH) for 3 weeks. The content of sodium picosulfate was evaluated. The content of sodium picosulfate was determined by 'Sodium picosulfate powder for oral solution' test method of *British Pharmacopoeia* using high performance liquid chromatography. The results were shown in Table 4 below.

**[Table 4]**

| Content (%) | Final pH | Initial | After 3 weeks Room temp. | After 3 weeks Stress condition |
|---|---|---|---|---|
| No pH controller | pH 6.7 | 102.1 | 101.8 | **2.0** |
| sodium hydrogencarbonate 0.25g / 3 00mL | pH 9.2 | 100.5 | 101.7 | 99.2 |
| sodium hydrogencarbonate 0.5g / 300mL | pH 9.8 | 102.1 | 101.7 | 100.2 |
| sodium hydrogencarbonate 0.75g / 300mL | pH 10.4 | 102.5 | 102.6 | 104.9 |
| sodium hydrogencarbonate 1.5g / 300mL | pH 11 | 103.0 | 103.6 | 105.7 |
| sodium citrate 1.5g / 300mL | pH 8.7 | 103.5 | 103.7 | 106.9 |
| sodium citrate 3g / 300mL | pH 8.9 | 104.7 | 104.5 | 111.2 |
| sodium citrate 4.5g / 300mL | pH 9.1 | 107.6 | 110.4 | 117.5 |

As shown in Table 4 above, sodium picosulfate's instability caused by mixing with polyethylene glycol was improved dramatically by comprising sodium hydrogencarbonate or sodium citrate.

### Experimental Example 3

0.01g of sodium picosulfate, 150g of polyethylene glycol 3350, 54.6g of sorbitol, and the following amount of sodium hydrogencarbonate were dissolved with heat in purified water and the total volume was 300 ml. The solution was stored at room temperature, in accelerated condition (40°C, 75%RH), and stress condition (60°C, 75%RH). The content of sodium picosulfate was evaluated. The content of sodium picosulfate was determined as mentioned in Experimental Example 2. The results were shown in Table 5 below.

**[Table 5]**

| Content (%) | Initial | After 5 weeks Room temp. | After 5 weeks Accelerated condition | After 5 weeks Stress condition |
|---|---|---|---|---|
| sodium hydrogencarbonate 0.25g / 300mL | 100.5 | 100.7 | 100.0 | 99.2 |
| sodium hydrogencarbonate 0.5g / 300mL | 102.1 | 101.7 | 100.2 | 99.0 |
| sodium hydrogencarbonate 0.75g / 300mL | 100.3 | 100.0 | 98.0 | 99.4 |

As shown in Table 5 above, sodium picosulfate was kept stable in the compositions according to the present invention.

## Claims

1. A purgative composition comprising polyethylene glycol and sodium picosulfate, further comprising a pH controller in order to improve the stability of sodium picosulfate.

2. The purgative composition of Claim 1, wherein the pH controller is an alkalizer.

3. The purgative composition of Claim 2, wherein the alkalizer is sodium hydroxide, sodium phosphate, sodium hydrogencarbonate, sodium citrate, potassium citrate, potassium hydroxide, potassium phosphate, magnesium carbonate, sodium carbonate, ammonium carbonate, or a mixture thereof.

4. The purgative composition of Claim 3, wherein the alkalizer is sodium hydrogencarbonate.

5. The purgative composition of Claim 1, wherein the composition is liquid and its pH is 7 to 12.

6. The purgative composition of Claim 1, wherein the composition is dissolved in purified water to make its total volume 300 ml and the pH of the prepared solution is 7 to 12.

7. A method for cleansing bowel, using a liquid purgative composition comprising 100-200g of polyethylene glycol having number average molecular weight of 3000-5000, 40-70g of sorbitol, 0.005-0.03g of sodium picosulfate, 0.1-0.4g of alkalizer, and a suitable amount of sweetener in 300 ml of solution,
wherein the method comprises (1) administering 100 to 200 ml of the purgative composition together with 300 to 600 ml of water, and then additional 400 to 600 ml of water within 30 minutes, and (2) administering 100 to 200 ml of the purgative composition together with 300 to 600 ml of water and then additional 400 to 600 ml of water within 30 minutes.

8. The method of Claim 7, wherein the alkalizer is sodium hydrogencarbonate.

9. The method of Claim 7, wherein the method is (1) administering 150 ml of the purgative composition together with 350 ml of water within 30 minutes, and then additional 500 ml of water within 30 minutes, and (2) after at least 1 hour, administering 150 ml of the purgative composition together with 350 ml of water within 30 minutes and then additional 500 ml of water within 30 minutes.
